# EUROPEAN PATENT APPLICATION

(11) **EP 2 027 868 A1**
(43) Date of publication of application: **25.02.2009**
(21) Application number: 07301323.7
(22) Date of filing: 24.08.2007
(51) Int. Cl.: A61K 38/10, A61K 38/17, A61P 25/28

(54) **Use of SCO-Spondin peptides for inhibiting or preventing neuronal apoptosis mediated by cell death receptor ligands**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Warcoin, Jacques

(57) **Abstract**

The invention relates to a polypeptide derived from the TSR (thrombospondin type 1 units) of SCO-Spondin for inhibiting or preventing the apoptosis mediated by the cell death receptor ligands, such as TRAIL or FasL. The polypeptide of the invention comprises a sequence -W-S-A1-C-S-A2-C-G- wherein A1 and A2 are amino acid sequences comprising 1 to 5 amino acids. More particularly, the invention relates to said polypeptide for inhibiting or preventing the apoptosis associated with a disease selected from the group consisting of neurodegenerative disorders, cerebral ischemia, neuronal traumas, neuronal inflammatory diseases, and viral neurodegenerations.

SEQUENCE LISTING

<110> Institut National de la Santé et de la Recherche Médicale
      (INSERM)

<120> A polypeptide for inhibiting or preventing neuronal
      apoptosis mediated by the cell death receptor ligands

<130> D25830

<160> 8

<170> PatentIn version 3.3

<210> 1
<211> 8
<212> PRT
<213> Artificial sequence

<220>
<223> Peptide derived from the TSR (thrombospondin type 1 units)
      of SCO-spondin

<220>
<221> MISC_FEATURE
<222> (3)..(3)
<223> Amino acid sequence comprising 1 to 5 amino acids

<220>
<221> MISC_FEATURE
<222> (6)..(6)
<223> Amino acid sequence comprising 1 to 5 amino acids

<400> 1

Trp Ser Xaa Cys Ser Xaa Cys Gly
1               5


<210> 2
<211> 9
<212> PRT
<213> Artificial sequence

<220>
<223> Peptide derived from the TSR (thrombospondin type 1 units)
      of SCO-spondin

<400> 2

Trp Ser Pro Cys Ser Val Thr Cys Gly
1               5


<210> 3
<211> 9
<212> PRT
<213> Artificial sequence

<220>
<223> Peptide derived from the TSR (thrombospondin type 1 units)

of SCO-spondin

<400> 3

Trp Ser Ser Cys Ser Val Thr Cys Gly
1               5


<210> 4
<211> 9
<212> PRT
<213> Artificial sequence

<220>
<223> Peptide derived from the TSR (thrombospondin type 1 units)
      of SCO-spondin

<400> 4

Trp Ser Gln Cys Ser Val Thr Cys Gly
1               5


<210> 5
<211> 4
<212> PRT
<213> Artificial sequence

<220>
<223> Sequence A1

<220>
<221> MISC_FEATURE
<222> (1)..(1)
<223> Amino acid chosen from the group consisting of G, S and C

<220>
<221> MISC_FEATURE
<222> (3)..(4)
<223> Amino acid chosen from the group consisting of G, S and C

<400> 5

Xaa Trp Xaa Xaa
1


<210> 6
<211> 4
<212> PRT
<213> Artificial sequence

<220>
<223> Sequence A1


<220>
<221> MISC_FEATURE
<222> (1)..(1)
<223> amino acid chosen from the group consisting of G, S and C

<220>
<221> MISC_FEATURE
<222> (4)..(4)
<223> amino acid chosen from the group consisting of G, S and C

<400> 6

Xaa Trp Ser Xaa
1


<210> 7
<211> 11
<212> PRT
<213> Artificial sequence

<220>
<223> Peptide derived from the TSR (thrombospondin type 1 units)
      of SCO-spondin

<220>
<221> MISC_FEATURE
<222> (3)..(3)
<223> amino acid chosen from the group consisting of G, S and C

<220>
<221> MISC_FEATURE
<222> (6)..(6)
<223> amino acid chosen from the group consisting of G, S and C

<220>
<221> MISC_FEATURE
<222> (9)..(9)
<223> amino acid sequence comprising 1 to 5 amino acids

<400> 7

Trp Ser Xaa Trp Ser Xaa Cys Ser Xaa Cys Gly
1               5                   10


<210> 8
<211> 12
<212> PRT
<213> Artificial sequence

<220>
<223> Peptide derived from the TSR (thrombospondin type 1 units)
      of SCO-spondin

<400> 8

Trp Ser Gly Trp Ser Ser Cys Ser Arg Ser Cys Gly
1               5                   10

## Description

### FIELD OF THE INVENTION:

The invention relates to a polypeptide derived from the TSR (thrombospondin type 1 units) of SCO-Spondin for inhibiting or preventing the neuronal apoptosis mediated by the cell death receptor ligands, such as TRAIL or FasL. More particularly, the invention relates to said polypeptide for inhibiting or preventing the neuronal apoptosis associated with a disease selected from the group consisting of neurodegenerative disorders, cerebral ischemia, neuronal traumas, neuronal inflammatory diseases, and viral neurodegenerations.

### BACKGROUD OF THE INVENTION:

Selective neuronal cell loss by apoptosis is a common feature of chronic neurodegenerative diseases such as Alzheimer's disease, amyotrophic lateral sclerosis, Parkinson's disease and Huntington's disease (Honig L.S. et al., 2001). Although necrosis has been considered as the dominant type of cell death in acute or subacute pathologic conditions induced by ischemia, toxins, trauma and infection, growing evidence suggests that apoptosis contributes significantly to neuronal loss in these processes.

Whatever the underlying cause, all neuronal apoptotic events share common features and involve a prescribed set of factors that include c-Jun N-terminal kinase (JNK), which activates c-Jun and thereby increases transcription of various apoptotic factors. Additionally, the intrinsic mitochondrial death pathway involving Bax, Apaf-1, caspase 9, and caspase 3 has been shown to be critical for apoptosis in both developing and mature injured neurons. Bax, a bcl-2 family protein, is of particular importance in neuronal apoptosis. When overexpressed, Bax promotes cell death (Oltvai et al. 1993), and Bax deficient sympathetic neurons deprived of NGF do not undergo normal apoptosis (Deckwerth et al. 1996). Other Bcl-2 family members are also important in modulating neuronal apoptosis, e.g. phosphorylation of Bim by JNK potentiates Bax-dependent apoptosis. (See, e.g., Putcha et al. 2001).

The apoptotic process can be triggered by either intrinsic or extrinsic signals (Cifone M.G.et al. 1995; Li H. et al. 1999.). The best example of the extrinsic pathway of apoptosis is the tumor necrosis factor (TNF) superfamily of cytokines such as TNF-α, Fas ligand (FasL) and TNF-related apoptosis inducing ligand (TRAIL). For example, FasL has been reported to be associated to a variety of neurologic disorders such as amyotrophic lateral sclerosis (Yi et al., 2000), Alzheimer disease (Su et al., 2003), and neuronal apoptosis following ischemia (Rosenbaum et al., 2000). Activation of death receptor signalling pathways was also detected during Parkinson disease (Hartmann et al., 2002). The function of other death receptors such as TRAIL receptors, also expressed in neurons, was detected during the neuronal degeneration occurring in several pathological conditions: inflammatory processes such as multiple sclerosis (Aktas et al., 2005), experimental models of neurodegeneration (Cantarella et al., 2003; Murata et al., 2006), Alzheimer patients (Uberti et al., 2004) and ischemia (Martin-Villalba et al., 1999).

Therefore methods that inhibit or prevent neuronal apoptosis would be particularly beneficial to those susceptible to suffer from neuronal injury or neurodegenerative disease.

The polypeptides of the invention have been described in the international patent application WO99/03890, as well as in Monnerie H. et al. (1998) and Meiniel et al. (2003). The polypeptides have been known up to now for their properties to increase neuritic growth (including the axons) in the cerebral cortex neurons in cases where cell degeneration has already occurred, such as in patients suffering from neurodegenerative disorders, neuronal injuries or traumas, etc...

However, no role has been suspected nor suggested for their ability to prevent neuronal apoptosis such as in neurodegenerative disorders, cerebral ischemia, neuronal traumas, neuronal inflammatory diseases and viral neurodegenerations in populations at risk. Populations at risk include people which are susceptible to suffer from such disorders, such as for example healthy members of a family where Alzheimer's disease has already occurred or in areas where a virus inducing neurodegeneration could spread. In such cases, there is a need for a medicament able to inhibit the neuronal apoptosis and consequently to prevent such diseases. The present invention provides a solution to this need by demonstrating that the polypeptides described herein are able to act on neuronal apoptosis mediated by the cell death receptor ligands (e.g., TRAIL, FasL).

### SUMMARY OF THE INVENTION:

The present invention relates to a polypeptide comprising a sequence:
-W-S-A1-C-S-A2-C-G-
wherein A1 and A2 are amino acid sequences comprising 1 to 5 amino acids for inhibiting or preventing the neuronal apoptosis mediated by the cell death receptor ligands.

The invention also relates to the polypeptide as above defined for inhibiting or preventing the apoptosis associated with a disease selected from the group consisting of neurodegenerative disorders, cerebral ischemia, neuronal traumas, neuronal inflammatory diseases, and viral neurodegenerations.

### DETAILED DESCRIPTION OF THE INVENTION:

The inventors have demonstrated that a peptide derived from the TSR (thrombospondin type 1 units) of the SCO-spondin inhibits the neuronal apoptosis mediated by the cell death receptor ligands (TRAIL, FasL...).

Therefore, a first object of the invention relates to the use of a polypeptide comprising or consisting of the following sequence
-W-S-A1-C-S-A2-C-G- (SEQ ID N°1)
wherein A1 and A2 are amino acid sequences comprising 1 to 5 amino acids, for the manufacture of a medicament for inhibiting or preventing the neuronal apoptosis mediated by at least one cell death receptor ligand.

A further object of the invention relates to a polypeptide comprising or consisting of the following sequence
-W-S-A1-C-S-A2-C-G- (SEQ ID N°1)
wherein A1 and A2 are amino acid sequences comprising 1 to 5 amino acids, for inhibiting or preventing the neuronal apoptosis mediated by at least one cell death receptor ligand.

It should be recalled that in the description as a whole, "amino acid" is understood to mean both the natural amino acids and the non-natural amino acids. "Natural amino acid" is understood to mean the amino acids in the L form which can be found in natural proteins, that is to say alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y) and valine (V). However, the present invention also relates to the non-natural amino acids, that is to say the preceding amino acids in their D form, as well as the homo forms of some amino acids such as arginine, lysine, phenylalanine and serine or the nor forms of leucine or valine.

In a particular embodiment, the cell death receptor ligand is FasL and/or TRAIL.

Advantageously, the neuronal apoptosis is associated with a disease selected from the group consisting of neurodegenerative disorders, cerebral ischemia, neuronal traumas, neuronal inflammatory diseases, and viral neurodegenerations.

The term "neuronal apoptosis associated with a disease" refers to the neuronal apoptosis which results from said disease.

In a particular embodiment, neurodegenerative disorders include but are not limited to amyotrophic lateral sclerosis (ALS), spinal muscular atrophy (SMA), Huntington's disease, Parkinson's disease, Alzheimer's disease, Diffuse Lewy Body disease, prion disease, progressive supranuclear palsy, multiple system atrophy, adrenoleukodystrophy, down syndrome and fronto-temporal dementia.

In another embodiment, the cerebral ischemia may be focal or global.

In another embodiment, neuronal traumas include but are not limited to anterograde degeneration, traumatic brain injury, spinal cord injury, and cholinergic denervation.

In another embodiment, neuronal inflammatory diseases include but are not limited to multiple sclerosis (MS), encephalomyelitis, HTLV-associated myelopathy, and meningitis.

In another embodiment, viral neurodegenerations include but are not limited to HIV-1 associated dementia, and neurodegenerations associated with moloney meurine leukemic virus, Borna disease virus, and Theiler's virus.

The invention also relates to the polypeptide of the invention for preventing ageing.

In a particular embodiment, A1 of SEQ ID N°1 is P.

In another particular embodiment A1 denotes the peptide consisting of the sequence X1-W-X2-X3 (SEQ IDN °5) wherein X1, X2, X3 are chosen, independently of each other, from the group consisting of G, S and C.

In another particular embodiment A1 denotes the peptide consisting of the sequence X1-W-S-X3 (SEQ ID N°6) and A2 is chosen from R-S, V-S and V-T.

In another particular embodiment, the polypeptide of the invention comprises or consists of the following sequence -W-S-X1-W-S-X2-C-S-A2-C-G- (SEQ ID N°7).

In another particular embodiment, the polypeptide of the invention comprises or consists of the sequence -W-S-G-W-S-S-C-S-R-S-C-G- (SEQ ID N°8).

The polypeptide of the invention may also comprise or consist in a sequence selected from the group consisting of the sequences -W-S-P-C-S-V-T-C-G- (SEQ ID N°2), -W-S-S-C-S-V-T-C-G- (SEQ ID N°3) and -W-S-Q-C-S-V-T-C-G- ( (SEQ ID N° 4).

Polypeptides of the invention may be produced by any technique known per se in the art, such as without limitation, any chemical, biological, genetic or enzymatic technique, either alone or in combination(s).

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the invention can be synthesized by recombinant DNA techniques as is now well-known in the art. For example, these fragments can be obtained as DNA expression products after incorporation of DNA sequences encoding a recombinant protein comprising the desired polypeptide into expression vectors and introduction of such vectors into suitable eukaryotic or prokaryotic hosts that will express the recombinant protein comprising the desired polypeptide, from which they can be later isolated using well-known techniques.

Polypeptides of the invention can be use in an isolated (e.g., purified) form or contained in a vector, such as a membrane or lipid vesicle (e.g. a liposome).

Alternatively, the invention relates to the use of a nucleic acid construct encoding a polypeptide as defined above for the manufacture of a medicament for inhibiting or preventing the neuronal apoptosis mediated by at least one cell death receptor ligand such as TRAIL and/or FasL. Advantageously, the neuronal apoptosis is associated with a disease selected from the group consisting of neurodegenerative disorders, cerebral ischemia, neuronal traumas, neuronal inflammatory diseases, and viral neurodegenerations.

The invention also relates to a nucleic acid construct encoding a polypeptide of the invention for inhibiting or preventing the neuronal apoptosis mediated by at least one cell death receptor ligand such as FasL and/or TRAIL. Advantageously, the neuronal apoptosis is associated with a disease selected from the group consisting of neurodegenerative disorders, cerebral ischemia, neuronal traumas, neuronal inflammatory diseases, and viral neurodegenerations.

In a particular embodiment, the invention pertains to a vector comprising a nucleic acid construct of the invention for inhibiting or preventing neuronal apoptosis induced by at least one cell death receptor ligand such as FasL and/or TRAIL. Adavantageously, the neuronal apoptosis is associated with a disease selected from the group consisting of neurodegenerative disorders, cerebral ischemia, neuronal trauma, neuronal inflammatory diseases, and viral neurodegenerations.

A further object of the invention relates to the use of a vector comprising a nucleic acid construct of the invention for the manufacture of a medicament intended to inhibit or prevent the neuronal apoptosis associated with a disease selected from the group consisting of neurodegenerative disorders, cerebral ischemia, neuronal traumas, neuronal inflammatory diseases, and viral neurodegenerations.

Such vectors/nucleic acid constructs may comprise regulatory elements, such as a promoter, enhancer, terminator and the like, to cause or direct expression of said polypeptide upon administration to a subject. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use.

Examples of plasmids include replicating plasmids comprising an origin of replication, or integrative plasmids, such as for instance pUC, pcDNA, pBR, and the like. Examples of viral vector include adenoviral, retroviral, herpesvirus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

The invention also relates to a polypeptide comprising or consisting of the following sequence
-W-S-A1-C-S-A2-C-G- (SEQ ID N°1)
wherein A1 and A2 are amino acid sequences comprising 1 to 5 amino acids, for preventing a disease selected from the group consisting of neurodegenerative disorders, cerebral ischemia, neuronal traumas, neuronal inflammatory diseases and viral neurodegenerations.

Advantageously, said neurodegenerative disorders are selected from the group consisting of amyotrophic lateral sclerosis (ALS), spinal muscular atrophy (SMA), Huntington's disease, Parkinson's disease, Alzheimer's disease, Diffuse Lewy Body disease, prion disease, progressive supranuclear palsy, multiple system atrophy, adrenoleukodystrophy, down syndrome and fronto-temporal dementia.

The invention also provides a method for inhibiting or preventing neuronal apoptosis associated with a disease selected from the group consisting of neurodegenerative disorders, cerebral ischemia, neuronal traumas, neuronal inflammatory diseases, and viral neurodegenerations, comprising administering a subject in need thereof with a therapeutically effective amount of a polypeptide or a nucleic acid construct or vector of the invention.

Also provided is a method for preventing a disease selected from the group consisting of neurodegenerative disorders, cerebral ischemia, neuronal traumas, neuronal inflammatory diseases, and viral neurodegenerations, comprising administering a subject in need thereof with a therapeutically effective amount of a polypeptide or a nucleic acid construct or vector of the invention.

By a "therapeutically effective amount" is meant a sufficient amount of said polypeptide or acid nucleic construct for inhibiting or preventing apoptosis at a reasonable benefit/risk ratio applicable to any medical treatment.

It is to be understood that the preferred polypeptides as defined above are also particular embodiments of these further aspects of the invention.

It will be understood that the total daily usage of the compounds and compositions of the present invention will be decided by the attending physician within the scope of sound medical judgment. The specific therapeutically effective dose level for any particular subject will depend upon a variety of factors including the disorder being prevented and the severity of the disorder; activity of the specific compound employed; the specific composition employed, the age, body weight, general health, sex and diet of the subject; the time of administration, route of administration, and rate of excretion of the specific compound employed; the duration of the prophylactic treatment; drugs used in combination or coincidental with the specific polypeptide employed; and like factors well known in the medical arts. For example, it is well within the skill of the art to start doses of the compound at levels lower than those required to achieve the desired prophylactic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject susceptible to suffer from the disorder. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The polypeptide and nucleic acid construct of the invention may be combined with pharmaceutically acceptable excipients, and optionally sustained-release matrices, such as biodegradable polymers, to form therapeutic compositions.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In a particular embodiment, polypeptides and acid nucleic constructs of the invention are intrathecally administered.

In another particular embodiment, polypeptides and acid nucleic constructs of the invention are delivered through a micro pump placed under the skin or directly in the brain. In such embodiment, polypeptides and acid nucleic constructs of the invention are delivered to where they are useful in a high amount.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The inhibitor of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject susceptible to suffer from the disorder. The person responsible for administration will, in any event, determine the appropriate dose for the individual subject.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

The compounds of the invention may be delivered in a pharmaceutically acceptable ophthalmic vehicle, such that the polypeptide can penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, inhibitors of the invention may be injected directly into the vitreous, aqueous humour, ciliary body tissue(s) or cells and/or extra-ocular muscles by electroporation means.

The pharmaceutical composition of the invention may comprise synthetic cerebrospinal fluid. An example synthetic cerebrospinal fluid is commercially available from ALZET Osmotic Pumps (Cupertino, CA, USA).

Inhibitors of the invention may also be combined with other anti-apoptotic agents to enhance their effectiveness. Such agents may include Rosiglitazone (Jung TW. et al. 2007).

The invention will be further illustrated in view of the following figures and examples.

### FIGURES:

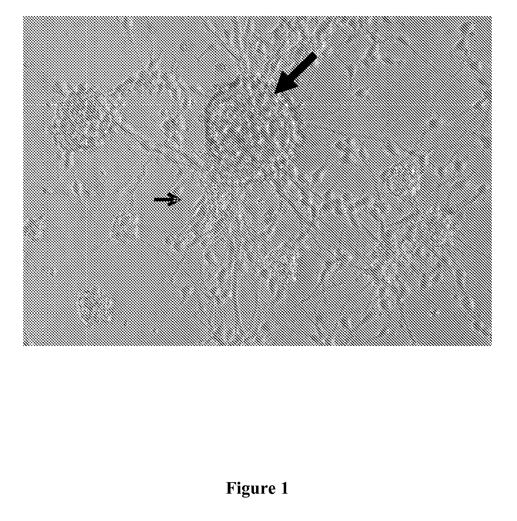

**Figure 1****: Photography of hippocampal embryonic cells at ED18 after 3 days in culture.** Cultures consist in low differentiated cells organized in clusters (neurospheres, large arrow), and migrating cells leaving clusters and beginning to differentiate (fine arrow).

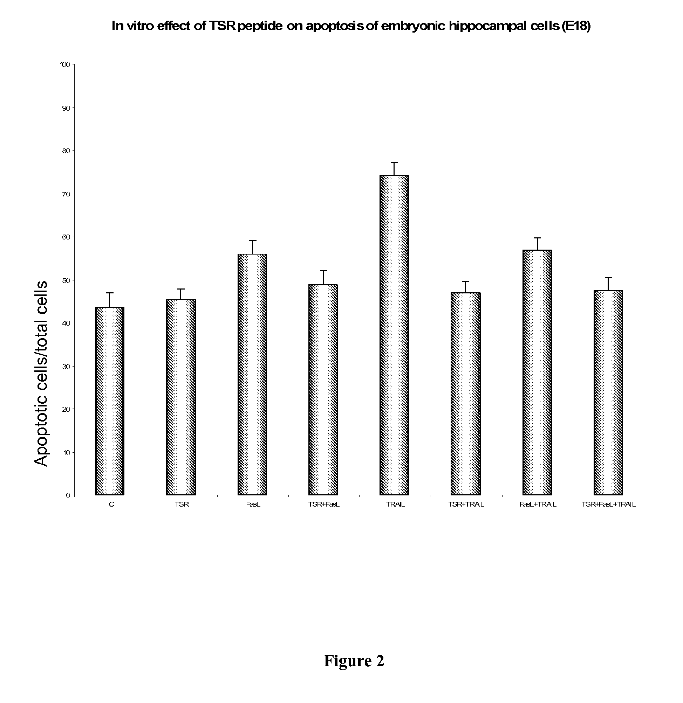

**Figure 2****: Histogramm representing the percentage of apoptotic cells outside the clusters compared with the total number of cells, data provided by only one** experiment realized in triplicate. In each assay, apoptotic labelling with TUNEL method were counted on 4 photos aleatory on each slide, and having always at least 1 neurosphere. The cultures were treated (cases TSR, FasL, TSR+FasL, TRAIL, TSR+TRAIL, FasL+TRAIL and TSR+FasL+TRAIL) or not treated (control, c) during 7 days with a TSR peptide concentration of 375 µg/mL and/or 20 ng/mL of FasL and/or 80 ng/mL of TRAIL).

### EXAMPLE:

### Material & Methods:

**Cultures of hippocampal cells from foetal rats:** Cells were obtained from E18 rat brains. Tissues were removed in dissection medium (Hank's Balanced Salt Solution, HBSS plus antibiotics). Composition of dissection medium HBSS plus antibiotics):
- potassium chloride (KCl): 400 mg/L
- potassium phosphate (KH2PO4): 60 mg/L
- sodium bicarbonate (NaHCO3): 350 mg/L
- sodium chloride (NaCl): 8000 mg/L
- sodium phosphate (Na2HPO4): 48 mg/L
- D-glucose: 1000 mg/L
- penicillin-streptomycin: 100 units/mL

Meninges were removed from the embryos brains and hippocampus was dissected in a sterile petri dish in HBSS plus antibiotic medium. After centrifugation cells were ressuspended in culture medium MEM with Earl Salts supplemented with B27, EGF, FGF-2 and antibiotics. The composition of the culture medium was the following :
- L-arginine hydrochloride: 126 mg/mL
- L-cystine: 24 mg/L
- L-glutamine: 292 mg/L
- L-histidine-H2O Hydrochloride: 42 mg/L
- L-isoleucine: 52 mg/L
- L-leucine 52 mg/L
- L-lysine Hydrochloride: 73 mg/L
- L-methionine: 15 mg/L
- L-phenylalanine: 32 mg/L
- L-threonine: 48 mg/L
- L-tryptophane: 10 mg/L
- L-tyrosine: 36 mg/L
- L-valine: 46 mg/L
- choline Chloride: 1 mg/L
- D-calcium pantothenate: 1 mg/L
- folic acid: 1 mg/L
- I-inositol: 2 mg/L
- Niacinamide: 1 mg/L
- pyridoxal Hydrochloride: 1 mg/L
- Riboflavine: 0,1 mg/L
- thiamine Hydrochloride: 1 mg/L
- calcium chloride (CaCl2-2H2O): 264 mg/L
- magnesium sulfate (MgSO4-7H2O): 200 mg/L
- potassium chloride (KCl): 400 mg/L
- sodium bicarbonate (NaHCO3): 2200 mg/L
- sodium chloride (NaCl): 6800 mg/L
- sodium phosphate (NaH2PO4-2H2O): 158 mg/L
- D-glucose: 4500 mg/L
- phenol red: 10 mg/L
- Sodium pyruvate: 110,04 mg/L
- B27 medium (Invitrogen) 50X: 2% v/v
- EGF: 20 µg/L
- FGF-2: 20 µg/L
- Penicillin-streptomycin: 100 units/mL

Hippocampus fragments were dissociated mechanically with 1 syringe 1 ml and 1 needle 25G and added with 1 mL of culture medium then centrifuged at 1200 rpm for 10 min. The supernatant is then removed and the cell pellet is resupended in 1 mL of culture medium. Other mechanic dissociation was performed with a seringe 1 mL and a needle 25G. Cell count was determined with Malassez hemocytometer and viability of cells was evaluated by counting the cells which have not absorbed the Trypan blue. Then cells are seeded in 48-well plates, at 40 000 cells by well in 500 µL of culture medium, on culture dishes precovered with collagen.

**Preparation of precovered dishes with collagen:** After an incubation of 10 min in absolute ethanol, the glass dishes were sterilized by heating 2H at 120°C. Sterilized glass dishes (10 mm diameter) was put on culture well and covered with a 0.1 mg/mL collagen calf skin solution. After 1 hr incubation at 37°C, the excess of solution is removed and the plate is maintained one night under hood of culture in order to let dry the plates. Before use, the wells were washed twice with 200 µL sterile distillated water.

**Treatment of embryonic rat hippocampal cells:** After 5 days of culture, cells were cultured in medium without growth factors (MEM with Earl salts, B27 and antibiotics) with different conditions of treatment combinating TSR peptide (WSGWSSCSRSCG - SEQ ID N°8) (375 µg/mL), FasL (20 ng/mL) and TRAIL (80 ng/mL). The composition of culture medium MEM, Earl salts, B27 and antibiotics is the following:
- L-arginine Hydrochloride: 126 mg/mL
- L-cystine: 24 mg/L
- L-glutamine: 292 mg/L
- L-histidine-H2O Hydrochloride: 42 mg/L
- L-isoleucine: 52 mg/L
- L-leucine: 52 mg/L
- L-lysine Hydrochloride: 73 mg/L
- L-methionine: 15 mg/L
- L-phenylalanine: 32 mg/L
- L-threonine: 48 mg/L
- L-tryptophane: 10 mg/L
- L-tyrosine: 36 mg/L
- L-valine: 46 mg/L
- choline Chloride: 1 mg/L
- D-calcium Pantothenate: 1 mg/L
- folic acid: 1 mg/L
- I-inositol: 2 mg/L
- Niacinamide: 1 mg/L
- pyridoxal hydrochloride: 1 mg/L
- Riboflavine: 0.1 mg/L
- thiamine hydrochloride: 1 mg/L
- calcium chloride (CaCl2-2H2O): 264 mg/L
- magnesium Sulfate (MgS04-7H2O): 200 mg/L
- potassium Chloride (KCl): 400 mg/L
- sodium bicarbonate (NaHCO3): 2200 mg/L
- sodium chloride (NaCl): 6800 mg/L
- sodium phosphate (NaH2PO4-2H2O): 158 mg/L
- D-glucose: 4500 mg/L
- phenol red: 10 mg/L
- Sodium Pyruvate: 110.04 mg/L
- B27 (composition : Invitrogen) 50X : 2% v/v
- Penicillin-streptomycin: 100 units/mL

**Apoptosis detection in treated hippocampal cells in cultures:** After a 7-day of treatment, the culture medium was removed and cells were fixed in a 4 % paraformaldehyde PBS solution (pH 7.6) during 30 min at RT and washed thrice with PBS. Cells were permeabilized with 0.2% de Triton X100-PBS solution at 4°C, for 5 min, washed thrice with PBS and maintained in equilibration buffer (10 min, RT).

### Equilibration buffer:

- potassium Cacodylate (pH6.6 at 25°C): 200mM
- Tris-HCl (pH6.6 at 25°C) : 25 mM
- Dithiothreitol (DTT): 0.2 mM
- Bovine serum albumin (BSA): 0.25 mg/L
- cobalt chloride: 2.5 mM

### rTdT Buffer:

- potassium cacodylate (pH6.6 at 25°C) : 176.47 mM
- Tris-HCl (pH6.6 at 25°C) : 22.06 mM
- Dithiothreitol (DTT) : 0.18 mM
- Bovine serum albumin (BSA) : 0.22 mg/L
- cobalt Chloride: 2.21 mM
- Fluorescein-12-dUTP : 4.9 µM
- dATP : 9.8 µM
- Tris-HCl (pH 7.6) : 0.98 mM
- EDTA : 98 µM
- Deoxynucleotidyl terminal transferase : 0.59 units/µL

The glass dishes containing cultures were incubated during 60 min at 37 °C with a drop of 25 µL of rTdT buffer following by incubation with 200 µL of SSC 2X/ well during 15 min at RT in the dark and three washes during 5 min with 200µL of PBS.

A counter-staining was performed with 4',6-Diamidino-2-phenylindole (DAPI), in order to determine in the same time the total cell number and to verify the number of apoptotic cells (with nuclear fragmentation) as a second evaluation. Cells were incubated during 5 min with 1 µg/mL of DAPI and washed thrice during 5 min with 200µL of PBS.

The glass dishes were mounted with Dako Fluorescent Mounting Medium (Dako), and stored at 4°C in the dark.

**Analysis of the results:** The results were photographed using a Leica microscope equipped with a digital camera coupled to a computer, at a rate of 4 photographs taken on each microscope slide. Each one of these photographs systematically contained 1 or several clusters of immature cells and was always carried out using the X10 objective. The total number of cells was evaluated by manual counting of the number of cells stained with DAPI. At the same time, the number of apoptotic cells was determined by counting of the number of TUNEL-positive cells, and correlated with the counting of DAPI stained cells exhibiting the nuclear fragmentation of apoptotic cells.

Statistical analyses were carried out by Statview 6 and Systat 10 software. Apoptosis values were compared using one way ANOVA. Values corresponded to mean ± standard error mean (SEM).

### Results:

At 3 days, cultures consist in clusters of undifferentiated cells, regarded as "neurospheres" and also migrating cells (Figure 1)).

After treatment during 7 days, apoptosis in hippocampal cells was decreased by peptide TSR treatment induced by cell death receptor pathway, as shown in Figure 1. The neuroprotective effect was dominant when apoptosis was induced by TRAIL.

The TSR peptide has no effect on the basal apoptosis (Figure 2). The number of apoptotic cells is increased in the presence of FasL compared to control cultures (Figure 2). Compared to FasL culture, the number of apoptotic cells is decreased in the presence of TSR peptide, demonstrating a protective effect of TSR peptide against the apoptosis induced by FasL (Figure 2). Furthermore, the number of apoptotic cells is more important than in the presence of FasL (Figure 2). Compared to TRAIL culture, the number of apoptotic cells is decreased in the presence of TSR peptide to a level comparable to control cells. TSR peptide has a protective effect on the TRAIL induced apoptosis (Figure 2). Finally, TSR peptide restores the basal level of apoptosis (Figure 2).

### Conclusions:

Our results show that the TSR peptide inhibits the apoptosis induced by the cell death receptors (Fas, TRAIL-R) when it was added in the culture medium of embryonic hippocampal cells.

### REFERENCES:

Aktas O, Smorodchenko A, Brocke S, Infante-Duarte C, Topphoff US, Vogt J, Prozorovski T, Meier S, Osmanova V, Pohl E, Bechmann I, Nitsch R, Zipp F. Neuronal damage in autoimmune neuroinflammation mediated by the death ligand TRAIL. Neuron. 2005 May 5;46(3):421-32.

Cantarella G, Uberti D, Carsana T, Lombardo G, Bernardini R, Memo M. Neutralization of TRAIL death pathway protects human neuronal cell line from beta-amyloid toxicity. Cell Death Differ. 2003 Jan;10(1):134-41.

Deckwerth TL, Elliott JL, Knudson CM, Johnson EM Jr, Snider WD, Korsmeyer SJ. BAX is required for neuronal death after trophic factor deprivation and during development. Neuron. 1996 Sep;17(3):401-11.

Ethell DW, Buhler LA. Fas ligand-mediated apoptosis in degenerative disorders of the brain. J Clin Immunol. 2003 Nov;23(6):439-46. Review.

Hartmann A, Mouatt-Prigent A, Faucheux BA, Agid Y, Hirsch EC. FADD: A link between TNF family receptors and caspases in Parkinson's disease. Neurology. 2002 Jan 22;58(2):308-10.

Honig L.S. and R.N. Rosenberg, Apoptosis and neurologic disease. Am. J. Med. 108 (2000), pp. 317-330; L.J. Martin, Neuronal cell death in nervous system development, disease, and injury (Review) . Int. J. Mol. Med. 7 (2001), pp. 455-478.

Jung TW, Lee JY, Shim WS, Kang ES, Kim SK, Ahn CW, Lee HC, Cha BS. Rosiglitazone protects human neuroblastoma SH-SY5Y cells against MPP+ induced cytotoxicity via inhibition of mitochondrial dysfunction and ROS production J. Neurol. Sci. 2007; 253:53-60.

Li H. and J. Yuan, Deciphering the pathways of life and death. Curr. Opin. Cell Biol. 11 (1999), pp. 261-266.).

Lovell MA, Geiger H, Van Zant GE, Lynn BC, Markesbery WR. Neurobiol Aging. 2006 Jul;27(7):909-17. Isolation of neural precursor cells from Alzheimer's disease and aged control postmortem brain.

M.G. Cifone, P. Roncaioli, R. De Maria, G. Camarda, A. Santoni, G. Ruberti and R. Testi, Multiple pathways originate at the Fas/APO-1 (CD95) receptor: sequential involvement of phosphatidylcholine-specific phospholipase C and acidic sphingomyelinase in the propagation of the apoptotic signal. EMBO J. 14 (1995), pp. 5859-5868

Martin-Villalba A, Herr I, Jeremias I, Hahne M, Brandt R, Vogel J, Schenkel J, Herdegen T, Debatin KM. CD95 ligand (Fas-L/APO-1L) and tumor necrosis factor-related apoptosis-inducing ligand mediate ischemia-induced apoptosis in neurons. J Neurosci. 1999 May 15;19(10):3809-17.

Meiniel A, Meiniel R, Goncalves-Mendes N, Creveaux I, Didier R, Dastugue B. The thrombospondin type 1 repeat (TSR) and neuronal differentiation: roles of SCO-spondin oligopeptides on neuronal cell types and cell lines. Int Rev Cytol. 2003;230:1-39.

Monnerie H, Dastugue B, Meiniel A. Effect of synthetic peptides derived from SCO-spondin conserved domains on chick cortical and spinal-cord neurons in cell cultures. Cell Tissue Research 1998 293, 407-418.

Murata T, Tsuboi M, Hikita K, Kaneda N. Protective effects of neurotrophic factors on tumor necrosis factor-related apoptosis-inducing ligand (TRAIL)-mediated apoptosis of murine adrenal chromaffin cell line tsAM5D. J Biol Chem. 2006 Aug 11;281(32):22503-16.

Oltvai ZN, Milliman CL, Korsmeyer SJ. Bcl-2 heterodimerizes in vivo with a conserved homolog, Bax, that accelerates programmed cell death. Cell. 1993 Aug 27;74(4):609-19.

Putcha GV, Moulder KL, Golden JP, Bouillet P, Adams JA, Strasser A, Johnson EM. Induction of BIM, a proapoptotic BH3-only BCL-2 family member, is critical for neuronal apoptosis. Neuron. 2001 Mar;29(3):615-28.

Rosenbaum DM, Gupta G, D'Amore J, Singh M, Weidenheim K, Zhang H, Kessler JA. Fas (CD95/APO-1) plays a role in the pathophysiology of focal cerebral ischemia. J Neurosci Res. 2000 Sep 15;61(6):686-92.

Steindler DA, Pincus DW. Stem cells and neuropoiesis in the adult human brain. Lancet. 2002 Mar 23;359(9311):1047-54. Review.

Tseng HC, Ruegg SJ, Maronski M, Messam CA, Grinspan JB, Dichter MA. Injuring neurons induces neuronal differentiation in a population of hippocampal precursor cells in culture. [Neurobiol Dis. 2006]

Uberti D, Cantarella G, Facchetti F, Cafici A, Grasso G, Bernardini R, Memo M. TRAIL is expressed in the brain cells of Alzheimer's disease patients. Neuroreport. 2004 Mar 22;15(4):579-81.

Yi FH, Lautrette C, Vermot-Desroches C, Bordessoule D, Couratier P, Wijdenes J, Preud'homme JL, Jauberteau MO. In vitro induction of neuronal apoptosis by anti-Fas antibody-containing sera from amyotrophic lateral sclerosis patients. J Neuroimmunol. 2000 Sep 22;109(2):211-20.

## Claims

1. Use of a polypeptide comprising the following sequence
-W-S-A1-C-S-A2-C-G- (SEQ ID N°1)
wherein A1 and A2 are amino acid sequences comprising 1 to 5 amino acids, for the manufacture of a medicament for inhibiting or preventing the neuronal apoptosis mediated by at least one cell death receptor ligand.

2. Use according to claim 1, wherein said cell death receptor ligand is TRAIL and/or FasL.

3. Use according to claim 1 or 2, wherein the neuronal apoptosis is associated with a disease selected from the group consisting of neurodegenerative disorders, cerebral ischemia, neuronal traumas, neuronal inflammatory diseases and viral neurodegenerations.

4. Use according to claim 3, wherein said neurodegenerative disorders are selected from the group consisting of amyotrophic lateral sclerosis (ALS), spinal muscular atrophy (SMA), Huntington's disease, Parkinson's disease, Alzheimer's disease, Diffuse Lewy Body disease, prion disease, progressive supranuclear palsy, multiple system atrophy, adrenoleukodystrophy, down syndrome and fronto-temporal dementia.

5. Use according to any of claims 1 to 4, wherein A1 is proline.

6. Use according to any of claims 1 to 4, wherein A1 denotes the peptide consisting of the sequence X1-W-X2-X3 (SEQ ID N°5) wherein X1, X2, X3 are selected, independently of each other, from the group consisting of G, S and C.

7. Use according to any of claims 1 to 6, wherein A2 denotes the peptide selected from the group consisting of R-S, V-S and V-T.

8. Use according to claim 6 or 7, wherein the polypeptide comprises the sequence consisting of -W-S-G-W-S-S-C-S-R-S-C-G- (SEQ ID N°8).

9. Use of a nucleic acid construct encoding a polypeptide as defined in any of claims 1 to 8 for the manufacture of a medicament for inhibiting or preventing the neuronal apoptosis associated with a disease selected from the group consisting of neurodegenerative disorders, cerebral ischemia, neuronal traumas, neuronal inflammatory diseases, and viral neurodegenerations.

10. Use of a polypeptide comprising the following sequence
-W-S-A1-C-S-A2-C-G- (SEQ ID N°1)
wherein A1 and A2 are amino acid sequences comprising 1 to 5 amino acids, for the manufacture of a medicament for preventing a disease selected from the group consisting of neurodegenerative disorders, cerebral ischemia, neuronal traumas, neuronal inflammatory diseases and viral neurodegenerations.

11. Use according to claim 10, wherein said neurodegenerative disorders are selected from the group consisting of amyotrophic lateral sclerosis (ALS), spinal muscular atrophy (SMA), Huntington's disease, Parkinson's disease, Alzheimer's disease, Diffuse Lewy Body disease, prion disease, progressive supranuclear palsy, multiple system atrophy, adrenoleukodystrophy, down syndrome and fronto-temporal dementia.
